# EUROPEAN PATENT SPECIFICATION

(11) **EP 3 682 428 B1**
(45) Date of publication and mention of the grant of the patent: **19.01.2022**
(21) Application number: 18855126.1
(22) Date of filing: 12.09.2018
(51) Int. Cl.: G07F 11/70, G07F 17/00

(54) **DEVICE FOR PACKAGING DOSED QUANTITIES OF SOLID MEDICINES**
VORRICHTUNG ZUM VERPACKEN DOSIERTER MENGEN VON FESTEN ARZNEIMITTELN
DISPOSITIF DE CONDITIONNEMENT DE QUANTITÉS DOSÉES DE MÉDICAMENTS SOLIDES

(30) Priority: 12.09.2017 NL 2019530
(43) Date of publication of application: 22.07.2020
(73) Proprietor: Canister Developments B.V., 3972 ET Driebergen-Rijsenburg (NL)
(72) Inventor: VAN WIJNGAARDEN, Arie, 3972 ET Driebergen-Rijsenburg (NL); VAN WIJNGAARDEN, Caroline, 3972 ET Driebergen-Rijsenburg (NL)
(74) Representative: Arnold & Siedsma
(86) International application number: PCT/NL2018/050600
(87) International publication number: WO 2019/054864

(56) References cited:
- EP-A1- 2 589 369
- EP-A1- 2 703 301
- EP-A1- 2 840 547
- EP-A2- 2 025 601
- WO-A1-98/29084
- US-A1- 2015 353 219

## Description

The present invention relates to an apparatus and method for packaging dosed quantities of solid medicines.

It is known, particularly in the case of chronically ill patients who are prescribed medicines, to provide the medicines in personalized packages, for instance in the form of medication bags and blister cards, wherein the medicine or several medicines which have to be taken at any given moment are combined in one package, for instance a medication bag or compartment of the blister card. Different bags or compartments of blister cards are mutually connected here and, in addition to the name and other personal data, clearly provided with an indication of the ingestion time. The bag or a compartment of the blister card is opened by the patient at the respective ingestion time and the medicines taken.

For filling of the personalized packages use is generally made of automated systems which comprise a plurality of canisters, each with a supply of medicines, and which are provided with a packaging device for packaging the medicines from the supplies in the canisters. Such systems are also referred to as automated dispensing cabinet (ADC), unit-based cabinets (UBCs), automated dispensing devices (ADDs), automated distribution cabinets or automated dispensing machines (ADMs).

On the basis of a prescription supplied to the system the system will control different canisters in order to dispense the prescribed medicines to the packaging device. The canisters in such a system are generally disposed above a collection tube or along a collection chute which is configured to receive and feed the medicines dispensed by the different canisters through to the packaging device. It is a drawback that contamination can occur in these systems, particularly in the collection tube and/or collection chute and the packaging device.

Another drawback is that medicines are in many cases supplied in blister format. Before the automated packaging of these medicines the medicines are removed, still in many cases manually, from the blister pack and collected in temporary storage jars/boxes or in the canisters for further processing. Filling of the canister with medicines from blister packs is therefore a labour-intensive process.

EP 2 025 601 A2 discloses a machine for handling blister packs, characterized in that it comprises a first blister pack storage station provided with an opening for feeding blister packs to a second blister pack transport station for transporting the blister packs to a third station for cutting the blister packs, and an opening for transferring the cut blister packs to a delivery or packing station.

WO 98/29084 A1 discloses a method and a device for automatic dispatching of singular items, specially an individual pill, from a storage station with several containers. Prepackaged, preselected flat item packagings ("blister") of the same type are piled in the form of substantially vertical storage columns in allocated containers. A blister packaging of a singular item to be dispatched is displaced to a withdrawing position in an area outside the pile, and a collecting shell is positioned thereunder. Subsequently, a withdrawing unit having a withdrawing finger which is allocated to the storage columns is positioned beneath the item to be dispatched which is in the withdrawing position. The withdrawing finger is moved downwards against an end support to separate the singular item from the rest of the blister packaging. The separated item falls by gravity in a predetermined cavity of the collecting shell. The item to be dispatched is separated by pressing out said item from the packaging with a pushing lug or by cutting out the item with a blade.

It is an object of the present invention, among others, to provide an apparatus for packaging dosed quantities of solid medicines wherein at least one of the above stated drawbacks is at least partially resolved, and/or to provide an efficient, reliable and/or improved apparatus for packaging dosed quantities of solid medicines.

This object is achieved, among others, with an apparatus according to claim 1. This object is more specifically achieved, among others, with an apparatus for packaging dosed quantities of solid medicines on the basis of a predetermined prescription, comprising:
- a control unit for controlling the apparatus and for receiving the prescription;
- a plurality of medicine holders, wherein a medicine holder is configured to hold a blister pack with medicines;
- a separating device for separating subject to the received prescription at least one of the medicines from a blister held in a medicine holder; and
- a packaging device configured to package the medicines separated by the separating device.

According to the invention the supplies of medicines, or at least a portion thereof, are held in the apparatus in blister form, either in blister pack or push-through strip. The individual medicines, also referred to below as medicine units, are therefore stored separated from each other in the apparatus, this minimizing the risk of contamination. It is moreover no longer necessary to remove the medicine units from a blister pack in order to fill a canister, as is necessary for the current packaging machines. This increases the efficiency of the apparatus.

The apparatus is provided with a separating device for separating one or more medicine units from the rest of a blister. The separating device is preferably controlled here for the purpose of dispensing the correct quantity of medicine units subject to the prescription. The apparatus preferably comprises a plurality of different types of medicine. On the basis of the prescription the correct type of medicine can then be selected with for instance a selection device and be separated into the correct quantity by the separating device. The separated medicines are then preferably guided, preferably with a suitable guide, to the packaging device. The guide is preferably a contamination-repellent guide, preferably by means of nanotechnology.

The prescription preferably comprises at least a list of the types of medicine and the number per medicine which must be packaged per package, for instance corresponding to an ingestion time for the patient. The control unit is then configured to separate, subject to the prescription, a predetermined quantity of medicines from a blister of a predetermined type of medicine. The packaging device is then preferably controlled, subject to this prescription, to package the medicines in a package, particularly once the predetermined number of medicines have been separated and dispensed to the packaging device. A package can therefore comprise different types of medicine, each in a different number, depending on the prescription.

The packaging device is configured to package in a single packaging the medicines separated in accordance with the prescription by the separating device. The packaging can for instance comprise a bag, wherein each bag contains a medicine or a plurality of medicines. The packaging device can for instance comprise a flow pack machine, wherein the individual batches of medicine are packaged in a single separate bag. Alternatively or additionally, the packaging device can be configured to package the medicines in a blister card, wherein the different batches of medicine are packaged in different blister compartments of a blister card. Blister cards and packaging devices herefor are per se known.

As discussed, holding the medicines in blister form has advantages in respect of for instance efficiency and reducing the risk of contamination, while this also increases the storage life of the medicines. Not every type of medicine is however suitable for packaging in blisters. A preferred embodiment of the apparatus therefore comprises additional dispensing devices for dispensing medicines subject to the control unit, for instance in the form of holders for receiving canisters and/or a drawer for the dispensing of medicines. The packaging device is then preferably configured to also package medicines coming from the dispensing devices under the control of the control unit. The dispensing devices are then preferably coupled via a suitable guide to the packaging device. The canisters can be standard canisters, or for instance canisters for broken or oddly shaped medicines as described for instance in the international patent publication WO 2017/003280.

According to a preferred embodiment of the apparatus, a medicine holder comprises a gripper which is configured to grip a blister pack. A gripper can clamp the blister packs in simple manner, in particular close to the edges and/or at the space between the medicines in the blister pack. The gripper is movable here between an open state, in which a blister pack can be brought within reach of the gripper in order to be gripped, and a closed state in which the blister pack is clampingly gripped.

A medicine holder, for instance in the form of the gripper, is preferably configured to hold a blister pack in a predetermined relative orientation such that the position of the blister pack, and thereby the medicines held therein as will be discussed in greater detail below, and the medicine holder is known. The separating device can then separate a medicine from the blister pack depending on the relative positions of a medicine holder and the separating device.

The medicine holders and the separating device are movable relative to each other, wherein the separating device is configured to separate medicines from blister packs which are held by a plurality of medicine holders. The separating device is configured here to separate medicines from a plurality of blister packs held in respective medicine holders. An efficient and compact apparatus is obtained by providing the apparatus with at least one separating device which is configured to successively separate medicines from blister packs held by different medicine holders. Subject to the prescription a predetermined type of medicine can then be selected, wherein the medicine holder holding the predetermined type of medicine and the separating device are moved toward each other in order to separate the predetermined type of medicine from the blister pack.

A compact and efficient apparatus is obtained as the separating device is disposed stationary in the apparatus, wherein the medicine holders are arranged movably for the purpose of carrying the blister packs held in the medicine holders toward and away from the separating device. In order to reduce the risk of contamination and to obtain an even more compact apparatus, according to a further preferred embodiment the separating device is disposed substantially above, or at least close to the packaging device, wherein the medicine holders are movable away from and toward the separating device. A medicine separated by the separating device can be efficiently guided here, preferably using only gravitational force, to the packaging device without further transport means being necessary for the purpose. The separating device is preferably disposed at or close to a central collection tube which extends in vertical direction and is preferably provided with contamination-repellent material, and which debouches in the packaging device.

An efficient control is obtained as the medicine holders are movable along a predetermined path, wherein the separating device is disposed along this path. The apparatus is then preferably provided with a suitable drive for carrying the medicine holders along the path. A further preferred embodiment also comprises an endless conveyor belt provided with the plurality of medicine holders, wherein the separating device is disposed along the path of the medicine holders, and a drive for driving the endless conveyor belt for the purpose of moving the medicine holders along the separating device. The plurality of medicine holders can thus be moved efficiently along the separating device.

According to a further preferred embodiment, the apparatus is provided with a plurality of separating devices with medicine holders associated therewith. The units of medicine holders and separating device can for instance be placed one above another in the apparatus. This increases the capacity of the apparatus. The separating devices are then preferably placed substantially vertically above one another on a central collecting device, for instance a tube, which preferably extends substantially vertically and which debouches in the packaging device. The different layers can then dispense medicines into the collection tube independently of each other for collection in the packaging device. A compact and efficiently controllable apparatus is obtained when it comprises a plurality of endless conveyor belts and separating devices associated therewith which are preferably placed above one another.

In order to further increase the capacity of the apparatus, the apparatus preferably comprises two or more groups of units of medicine holders and separating devices placed above one another, wherein the central collection tube is located between the two groups, wherein the separating devices are disposed on either side of the collection tube.

A further preferred embodiment comprises a housing in which at least the medicine holders are located, wherein the housing is provided with a climate control system for controlling the climate in the housing. The medicines are stored here in a climate-controlled environment. This increases the storage life of the medicines, in particular the medicines which are not used frequently. In the known systems, wherein the medicines are stored in jars or canisters after removal from the blister packs, the longer the medicines are kept in a non-conditioned space in storage jars or canisters, the shorter their storage life.

An extraction system is preferably further provided for discharging residual waste and dust particles. This minimizes the risk of contamination. The separating device and/or the packaging device and optional guides are preferably also situated in the housing. The consequences of possible dust formation in these components are then minimized. The climate control system is preferably configured here to hold the contents of the housing at an underpressure relative to the surrounding area. This minimizes the risk of contamination from this surrounding area. According to a further preferred embodiment, the apparatus further comprises an outlet for discharging residual material, for instance in the form of empty blister packs or erroneously separated medicines. The outlet is preferably situated close to the central collection tube and/or the buffer, wherein a suitable guide is provided for discharging the material subject to the instructions from the control unit. Preferably provided for the purpose of discharging erroneously separated medicines from the buffering devices are switching means which guide the erroneously separated medicines to the outlet. The apparatus preferably comprises a separate outlet for discharging empty or no longer required blister packs held in the medicine holders which can be provided in an end surface of the apparatus located opposite the central collection tube.

For an efficient and rapid processing of the prescription a further preferred embodiment of the apparatus further comprises a buffering device between a separating device and the packaging device for buffering separated medicines for dispensing to the packaging device. A subsequent set of medicines can meanwhile then be created at the buffer during packaging of a preceding set of medicines.

Particularly when use is made of a plurality of groups or units of separating devices with associated medicine holders, it is advantageous when medicines coming from different separating devices can be pre-collected or buffered with medicine holders associated therewith.

A further efficient processing is obtained when the apparatus comprises a first buffering device between a separating device and the central collection chute for buffering separated medicines for dispensing to the collection tube. This makes it possible to already pre-collect some different types of medicine, coming for instance from the same group of medicine holders associated with one separating device, for dispensing to the packaging device, optionally at the first buffer upstream thereof as explained above. In the case of a plurality of separating devices with associated medicine holders operating in parallel, different sets of medicines can be prepared in parallel in a buffer for later packaging in the packaging device.

An efficient buffering device is obtained when the buffering device comprises a movable body which is movable into and out of the passage, for instance of a guide for medicines, for receiving and buffering a medicine on the movable body. A suitable buffering device comprises a diaphragm provided with a plurality of movable blades.

In order to increase the reliability of the apparatus, according to a further preferred embodiment the apparatus also comprises a detection device for inspecting and preferably identifying the buffered medicine. The detection device, for instance in the form of a digital camera, is preferably configured to provide image information of the medicine, or plurality of medicines, in the first buffer. The image information can then be processed, for instance in the control unit, for the purpose of checking the dispensed medicines. The image processing can then comprise of counting the buffered medicines, wherein the number of buffered medicines can be checked on the basis of the prescription. It is however preferred that in addition to the number also the type of medicine can be identified, for instance by suitable image recognition software. The control unit, or perhaps another component of the apparatus, is then preferably configured to check the identified medicine on the basis of the prescription. A database of image material, or parameters derived therefrom, of a plurality of types of medicine can be provided here, on the basis of which a medicine in the provided image material can be identified and then preferably checked against the prescription.

It is preferred here that the inspection step takes place prior to the packaging, wherein the medicines which have to be packed in one packaging are held simultaneously in a second buffer for detection. The apparatus is configured here to inspect and identify a set of separated medicines for packaging in a single package. The complete content of a packaging can then be efficiently detected or inspected. An inspection step is then no longer necessary after packaging, this increasing the efficiency of the packaging process. In the present systems it is necessary to check with a separate monitoring system whether the correct tablets are in the correct packagings, this being a costly step.

At least one additional buffer is preferably provided downstream relative to the buffer where the visual inspection takes place and upstream relative to the packaging device. A more efficient apparatus is hereby obtained.

If medicines deviating from the prescription are detected, the control unit can generate a suitable output, for instance in the form of an alarm signal, a message on a display unit, for instance a screen, and/or cease the processing in the apparatus. When deviating medicines are detected, the medicines are preferably discharged via for instance the above described outlet. The control unit can then be configured to reprocess the erroneously implemented prescription.

According to a further preferred embodiment, the apparatus, preferably the control unit, comprises a memory which comprises position data, wherein the position data for the plurality of blister packs comprise the positions of medicines in the respective blister packs. For each blister pack held in the medicine holders the positions, and preferably also sizes, of the medicines in the respective blister pack are stored in the memory. When a blister pack is moved in a medicine holder to the separating device, the positions of the medicines in this blister pack can be retrieved from the memory. The position data preferably comprise not only the positions of the medicines in a starting situation, i.e. a full blister pack, but the data are also modified when a medicine is separated.

The separating device is then preferably configured to separate at least one of the medicines from the blister pack on the basis of the position data of the respective blister pack. The separating device and the blister pack can then be moved and aligned relative to each other for the purpose of separating the medicine defined according to the prescription.

In order to obtain the position data the apparatus preferably further comprises a detection device for detecting the positions of the medicines in a blister pack for the purpose of providing the position data. The detection device can make an image of the blister pack and, with suitable image processing, derive on the basis thereof the positions and preferably dimensions of the medicines in the blister pack. These data are then stored as position data. It is possible for the position data to be determined before separation, wherein the medicines are separated subject thereto. It is however preferred that the memory comprises position data of the blister packs held in the medicine holders so that separation can take place efficiently and without delay on the basis of already available position data.

According to a further preferred embodiment, the separating device is configured to press at least one medicine out of the blister pack. The medicine is taken out of the blister pack here. The separating device preferably comprises for this purpose a first body and clamping body, wherein the separating device is configured to press at least one medicine out between the first body and the clamping body. A medicine is separated efficiently by moving the bodies toward each other, wherein the first body for instance provides counter-pressure as the clamping body moves toward the first body.

By moving the bodies relative to the blister pack a medicine can be selected from the blister pack for separation. At least one of the first body and the clamping body are preferably moved relative to the blister pack depending on the position data of this blister pack for the purpose of separating at least one medicine by pressing it out. The blister pack is aligned here relative to the separating device on the basis of the position data such that the predetermined medicine unit can be pressed out by moving the bodies toward each other following alignment.

The position of the medicines in the blister pack and the position of the gripper, and thereby the blister pack, is preferably known in relation to the separating device, whereby the first body and the clamping body can be moved in a plane parallel to the blister pack until both bodies are aligned with the medicine to be pressed out. The apparatus is thus able to separate all medicines if required. In a further preferred embodiment it is also known which medicines in the blister pack have already been separated, so that the first body and the clamping body can be moved in a plane parallel to the blister pack to the next medicine to be separated in the blister pack when dispensing thereof is requested.

Efficient pressing-out takes place when the first body comprises an opening for receiving the medicine during movement of the clamping body toward the first body. The blister pack is then supported by the first body around the medicine to be separated while the clamping body presses the medicine out. The first body is preferably moved here on the basis of the position data for the purpose of aligning the medicine relative to the opening. The first body and the clamping body are preferably moved together on the basis of the position data for the purpose of aligning the medicine relative to the opening. This alignment then preferably takes place in a plane parallel to the plane of the blister pack (for instance an underside thereof), while the movement for pressing out a medicine preferably takes place in a direction perpendicularly of this plane.

Efficient pressing-out, wherein the risk of damage to the medicine is limited, takes place when the first body is provided with a milling device, preferably in the form of serrations, for cutting into the blister pack. The packaging will then open at the serrated edge, this providing for efficient pressing-out.

According to a further preferred embodiment, the separating device is provided with a cutting device for severing a part of the blister pack with the at least one medicine therein. Since specific medicines have to be held in a protective atmosphere, it is particularly advantageous to be able to dispense them in the requested numbers in the original packagings, whereby the medicines are not exposed for an unnecessarily long time to the outside air.

The cutting device preferably comprises a laser for cutting the blister packs. A particularly efficient and flexible cutting device is obtained when the laser beam is movable in the plane parallel to the held blister pack. It is advantageous that the position data, sizes and orientations of the medicines in the blister pack are determined prior to them being cut out. It is however preferred that the memory comprises position data, sizes and orientations of the blister packs held in the medicine holders so that a cutting line around the part of the blister pack to be cut out can be determined efficiently and without delay on the basis of these available data, and this part of the blister can be cut out by moving the laser beam over the defined cutting line.

It is noted that, although the separating devices as described above can be used particularly well in the apparatus as described above with for instance medicine holders movable along a path and a packaging device, the separating device per se can also be used in other directions, optionally with for instance a stationary medicine holder or manually operated medicine holder.

According to a preferred embodiment, the apparatus comprises at least one storage for holding a plurality of blister packs. The apparatus preferably comprises a plurality of storages, preferably at least one for each type of medicine. The storages can for instance be filled manually. The apparatus is preferably configured to a grip a blister pack in a storage with a medicine holder, for instance with the gripper as discussed above, and to move it to the separating device. The storages are preferably disposed for this purpose along the path of the medicine holders, optionally arranged in multiple layers as described above.

The storage preferably comprises a transport mechanism for carrying a blister pack to a medicine holder. The transport mechanism preferably guides the lowermost blister pack out of the storage to for instance the gripper on the endless conveyor belt at the moment that a new blister pack has to be loaded onto the gripper. For the stability of the blister pack on the gripper it is particularly advantageous when the gripper can grip symmetrically relative to the centre of the blister. This can be realized by providing the storage cabinet with a centring device which centres the blister packs in the storage cabinet, whereby they also lie centred relative to the gripper when this latter has to be provided with a new blister pack.

The invention moreover provides a method for packaging dosed quantities of solid medicines on the basis of a predetermined prescription, in particular with an apparatus according to the invention, wherein the method comprises the steps of:
- providing a plurality of blister packs with medicines and held in medicine holders, wherein a medicine holder is configured to hold a blister pack;
- selecting subject to the prescription at least one predetermined type of medicine from the plurality of medicines;
- separating a predetermined quantity of medicines from the selected medicine with a separating device; and
- packaging the separated medicines.

As described above, it is advantageous here that the selection comprises of moving one of the medicine holders which holds a blister pack with the predetermined type of medicine to the separating device. The movement preferably takes place using the endless conveyor belt as described above.

A preferred embodiment of the method also comprises the step of buffering a separated medicine prior to the step of packaging. The apparatus is preferably provided for this purpose with a buffering device as described above. The buffering device makes it possible to already prepare the medicine holders and the separating device for a subsequent step, and optionally to already separate and buffer a subsequent medicine in accordance with the prescription. For the purpose of checking the separated medicine the method further comprises the step of inspecting and identifying the buffered medicine and checking the identified medicine against the prescription. Using an inspection device it is therefore possible to ensure a correct medication for a patient as already elucidated above. The method preferably comprises of inspecting and identifying a set of separated medicines for packaging in a single package.

A further preferred embodiment of the method further comprises the step of providing position data, wherein position data for the plurality of blister packs comprise the positions of medicines in the respective blister packs, wherein separation comprises of separating at least one medicine subject to the positions of the medicines in the respective blister pack. As explained above, this enables an efficient separation.

According to a further preferred embodiment, separation comprises of pressing a medicine out between two bodies. At least one of the bodies is preferably moved subject to the position data for the purpose of separating the at least one medicine.

The present invention is further illustrated on the basis of the following figures which show a preferred embodiment of the apparatus according to the invention and are not intended to limit the scope of the invention in any way, wherein:
- Figures 1A and B show respectively a top and bottom view of a blister pack;
- Figure 2 shows an overview of the apparatus wherein a part of the apparatus is shown cutaway and so shows a part of the internal systems;
- Figure 3 shows a block diagram of the components in the apparatus;
- Figures 4A and B show respectively a partial front view and a top view of the medicine transport system;
- Figure 5 shows in perspective details of an endless conveyor belt with a medicine holder with blister pack;
- Figure 6 shows in perspective two endless conveyor belts with a medicine holder with blister pack, a separating device and a first buffering device;
- Figure 7 shows in perspective a press-out unit for pressing medicines out of the blister packs;
- Figure 8 shows a section along the line VIII in figure 7;
- Figure 9 is a perspective view of the pressing-out process;
- Figure 10 shows in perspective the underside of a blister pack, wherein a single medicine is being pressed out;
- Figure 11 shows in perspective a cutting device which makes use of a laser to cut out the blister packs to be singulated;
- Figure 12 shows in perspective the process of cutting out by means of a laser;
- Figures 13A and B show respectively a perspective front and rear view of a storage for blister packs;
- Figure 14 shows a storage in schematic cross-section along the line XIV in figure 13B;
- Figure 15 shows in perspective a first buffering device with inspection device;
- Figure 16 shows in perspective the second buffering device;
- Figure 17 shows a package with medicines; and
- Figure 18 shows a processing diagram for packaging medicines.

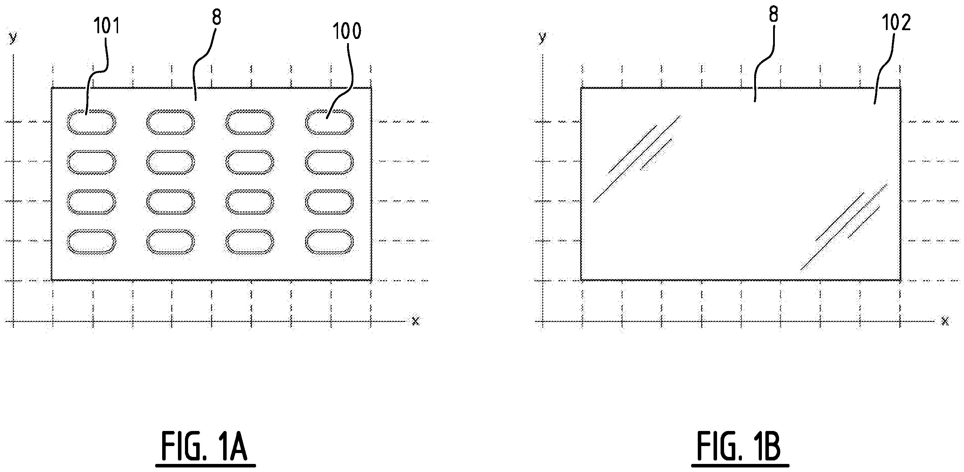

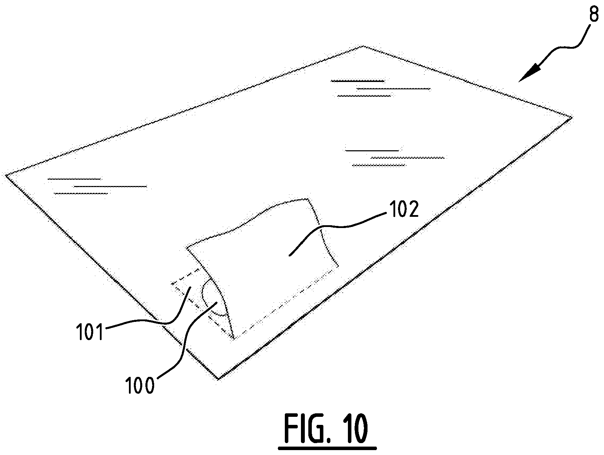

Figures 1A and B show respectively a top and bottom view of a blister pack 8 for medicines. Such a blister pack generally comprises one or more individually enclosed/packaged medicines 100. A blister pack comprises a first layer 101, in which a number of, in this example 16, holders for holding medicines 100 are arranged. The holders are closed by arranging a second layer 102 on first layer 101. First layer 101 is usually made of an optionally transparent plastic, while second layer 102 generally comprises a thin plastic or metal foil. A medicine can be taken out of blister pack 8 by pressing on the holders for medicines 100, as shown in figure 10.

An important aspect of the invention, as will be discussed in greater detail below, is that the location, indicated in this example with X and Y in the coordinate system, of medicines 100 in a blister pack is known.

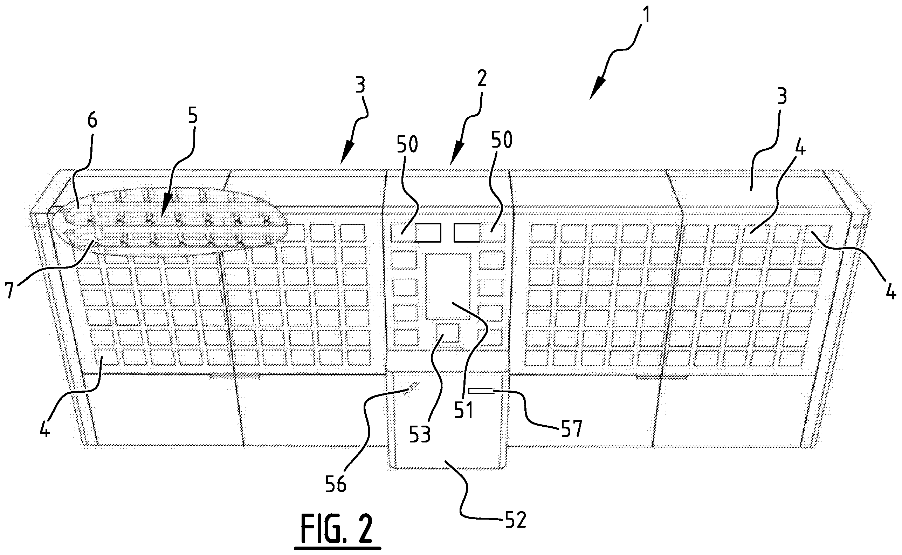

Figure 2 shows an apparatus 1 for packaging dosed quantities of solid medicines 100. The apparatus is constructed in this example from a central part 2, wherein cabinets 3 are placed on either side. Central part 2 is provided with a number of receiving spaces 50 in which medicine dispensing devices such as canisters can be placed. Larger canisters can also be placed in the central part by providing the apparatus with mutually adjacent receiving spaces. These can be seen on the upper part of central part 2. In order to reduce the drop height of the medicines coming from these canisters it can be advantageous to place these canisters lower in central part 2. Although not shown, apparatus 1 can moreover be provided, for instance in central part 2, with a receiving space for a drawer for holding and dispensing broken and/or oddly shaped medicines.

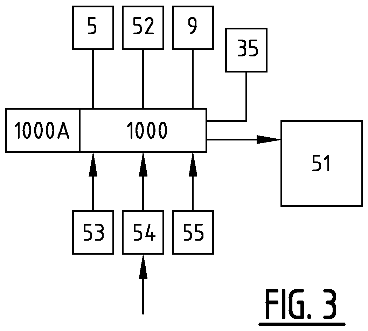

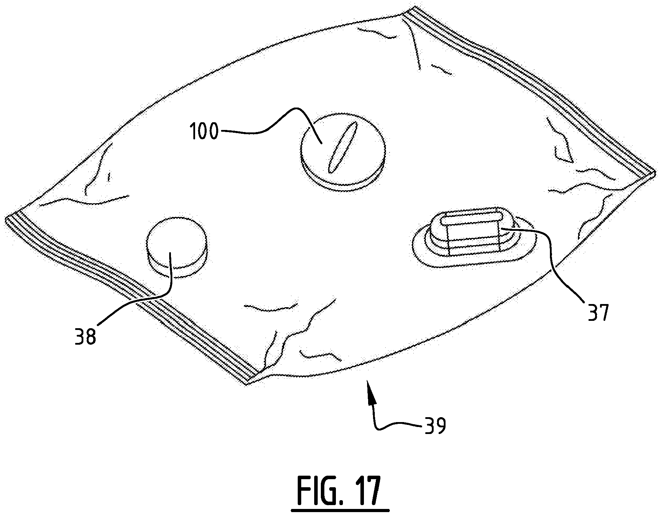

Central part 2 is moreover provided with a screen 51 for displaying the status of the apparatus. The screen can be embodied as touchscreen as input unit; alternatively or additionally a separate input unit 55 can be provided in the form of for instance a keyboard (indicated schematically in figure 3). Placed on the underside of central part 2 is a packaging device 52 which is configured to package batches of medicines. Such a packaging in the form of a bag is for instance shown in figure 17, for which an outlet 56 is provided in packaging device 52. Packaging devices for packaging medicines, for instance in the form of a flow pack machine, or for packaging medicines in blister cards are known as such. Packaging device 52 is in addition provided with a separate outlet 57 for blister cards.

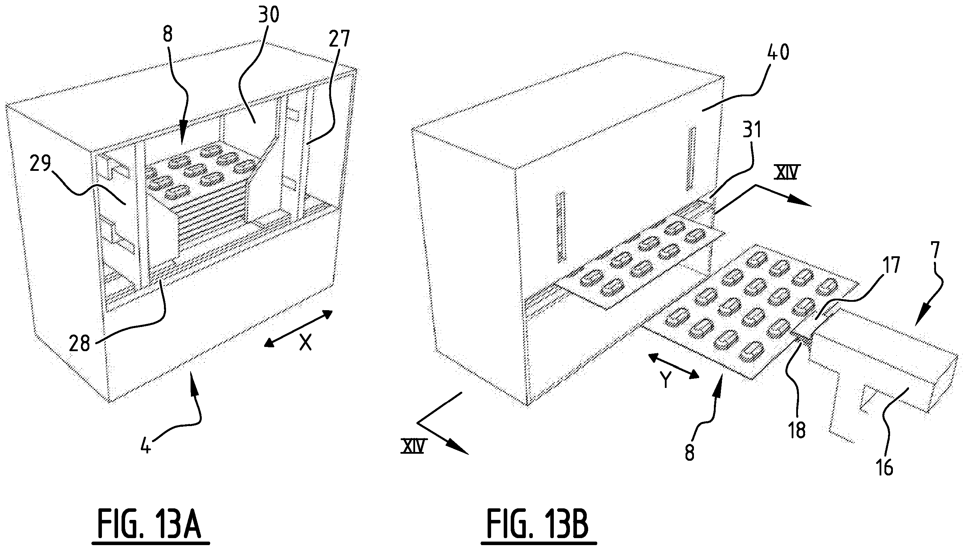

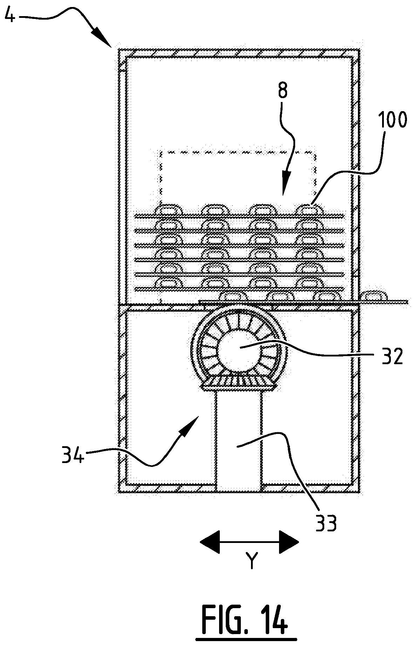

Cabinets 3 comprise on the front and rear sides a plurality of storages 4 in which medicines can be stored in blister form. A storage 4 is shown in greater detail in figures 13 and 14 and the construction will be discussed in greater detail below. Different types of medicine can be stored in storages 4 in order to be packaged in packaging device 52 on the basis of a prescription. Central part 2 is moreover provided with a scanning device 53 in which blister packs 8 can be scanned prior to filling of storages 4. This will be elucidated in greater detail below.

A central processing unit or control unit 1000 (figure 3), for instance in the form of computer, and preferably a memory 1000A thereof, is configured to store data relating to the type of medicine in each of the storages 4, which are for instance provided for this purpose with a unique identification number. Control unit 1000 is operatively connected to each of the components of the apparatus, such as display unit 51, input 55, scanning device 53. The control unit moreover controls the different components such as packaging device 52, separating devices 9 and medicine transport system 5. Also shown schematically in figure 3 is a receiver unit 54 which is configured to receive prescriptions. Receiver unit 54 is for instance configured to receive digital data from for instance a pharmacist system.

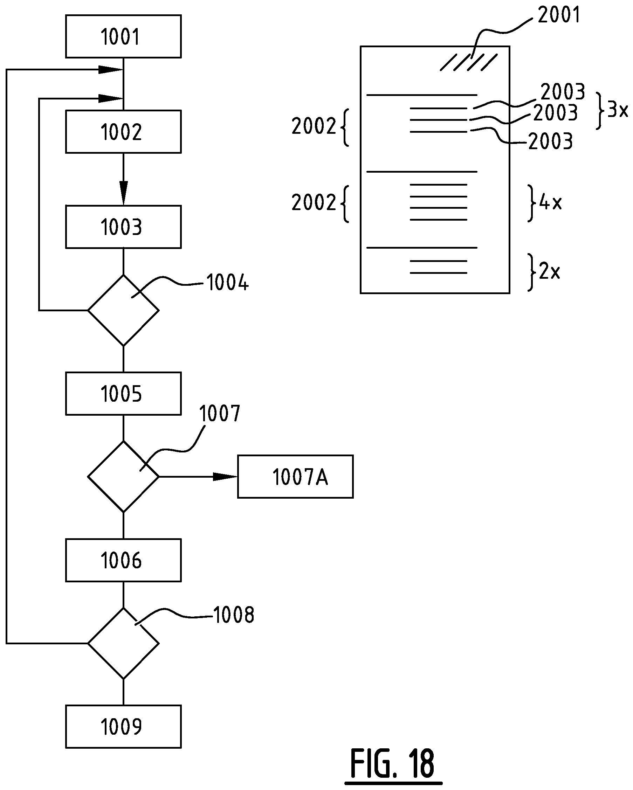

This prescription, which is preferably received in digital form and processed by control unit 1000 and an example of which is shown in figure 18, comprises a list of medicines which have to be packaged together in a single packaging. Those medicines which for instance have to be taken at one ingestion time are generally packaged together for a patient. Control unit 1000 is then configured to select these medicines, for instance from storages 4, on the basis of the prescription and provide them to packaging device 52 for packaging.

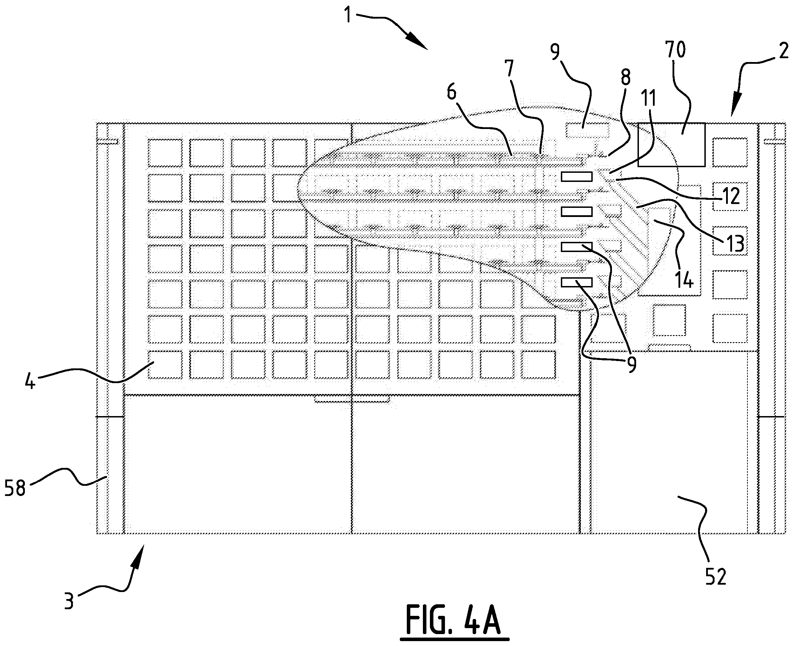

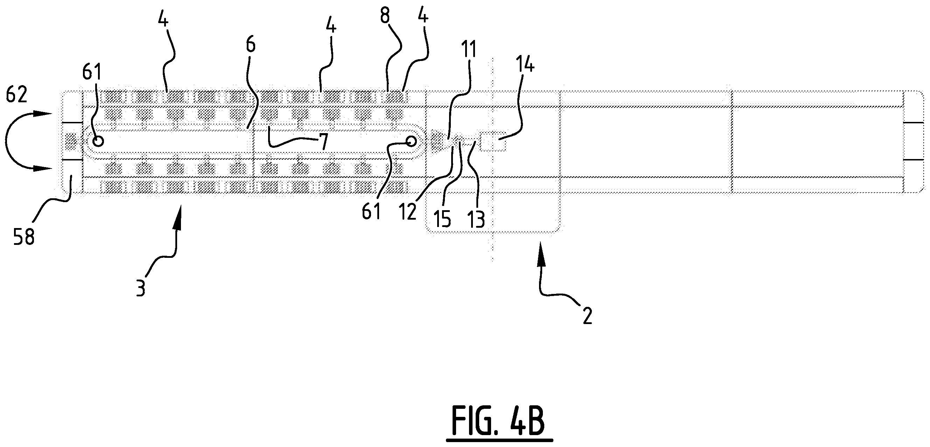

Apparatus 1 is configured to hold at least a portion of the medicines in the blister pack. Apparatus 1 is provided for this purpose with a plurality of transport systems 5 for selecting and transporting the medicines 100 in blister packs 8 to separating devices 9, see figures 4A and B. In this example seven separate transport systems 5 are arranged one above another per cabinet. A transport system 5 comprises an endless conveyor belt 6 on which a plurality of medicine holders in the form of grippers 7 are arranged. Conveyor belt 6 is driven by a drive which is designated schematically with reference numerals 61 in figure 4. Each transport system 5 in apparatus 1 can be individually controlled by control unit 1000.

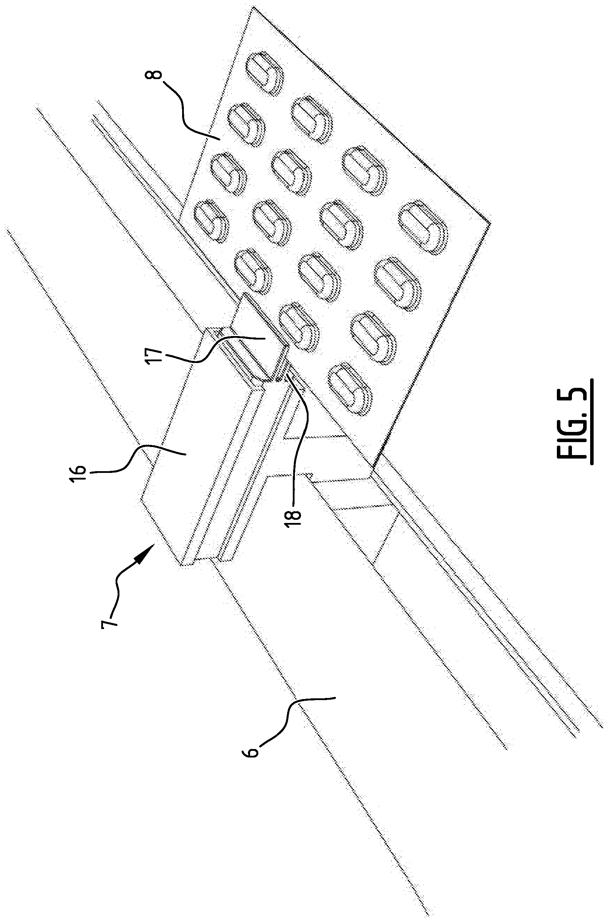

An individual medicine holder is shown more clearly in figure 5. Gripper 7 of a medicine holder is coupled to endless conveyor belt 6 such that, by driving conveyor belt 6, for instance with drives 61, the exact position of gripper 7 is known. In this example gripper 7 is coupled with a central body 16 to conveyor belt 6, wherein at an outer end a gripping mechanism is provided which is formed by a first clamping element 17 and a second clamping element 18. The gripping mechanism is configured to clamp a blister pack 8 fixedly therebetween, preferably such that the position of blister pack 8 relative to the whole of gripper 7, for instance central body 16 thereof, and thereby conveyor belt 6, is known. By driving conveyor belt 6 blister pack 8 can be carried in accurate manner along the path of endless conveyor belt 6, indicated schematically with arrow 62 in figure 4B.

In order to enable packaging of a predetermined number of medicines from a blister pack 8 on the basis of the prescription the apparatus 1 comprises separating devices 9. A separating device 9 is configured to separate, subject to the prescription, at least one of the medicines 100 from a blister pack 8 held in a medicine holder 7. In this example each transport system 5 in a layer is provided with separating device 9, and the separating devices are disposed vertically relative to each other above packaging device 52.

Control unit 1000 is configured here to store for each medicine holder 7 in the apparatus which type of medicine 100 is held in blister packs 8. When a specific type of medicine has to be packaged, control unit 1000 controls the respective transport system 5 and drives conveyor belt 6 such that medicine holder 7 with the specific type of medicine 100 therein is aligned with a separating device 9. Separating device 9 separates the desired number of medicines and feeds these to packaging device 52 for packaging.

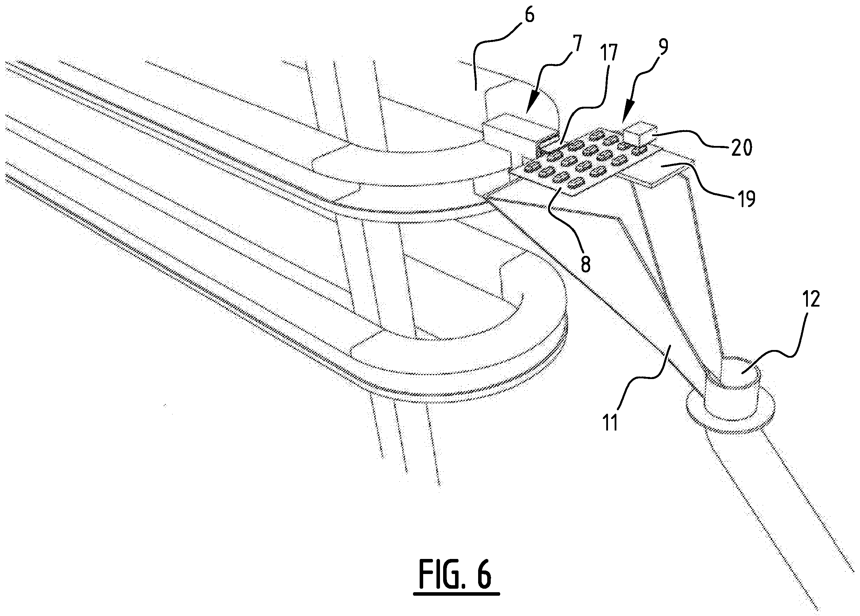

Provided under separating devices 9 for an efficient feed of the separated medicines to packaging device 52 are funnels 11 (see also figure 6) which guide the separated medicines to a first buffering device 12. First buffering device 12 is situated between funnel 11 and connecting tube 13, which is in turn connected to a central collection tube 14. As discussed above, the canisters in receiving spaces 50 also debouch, directly or indirectly, into collection tube 14 so that, subject to the control by control unit 1000, medicines can also be guided out of the canisters via collection tube 14 to packaging device 52.

Referring to figure 4, a climate control unit 70 which controls the climate in the housing of apparatus 1 is provided on the upper side of central part 2. Climate control unit 70 cools the interior of the apparatus so that the medicines held in storages 4 and in the medicine holders are stored cooled. Climate control unit 70 comprises an extraction system for extracting possible dust resulting from guiding and/or separation of the medicines. The interior of apparatus 1 is moreover kept at an underpressure relative to the surrounding area in order to prevent contamination of this surrounding area. Outlet 58 for discharging empty or no longer required blister packs 8 held in medicine holders 7 is further also provided in cabinet 3. As soon as a blister pack is empty or if for instance more medicine units are required for a single packaging than still remain in the blister pack, this is carried to the discharge station on the end surface of the cabinet and released by gripper 7, after which the discharged blister packs accumulate at the bottom of cabinet 3 in outlet 58.

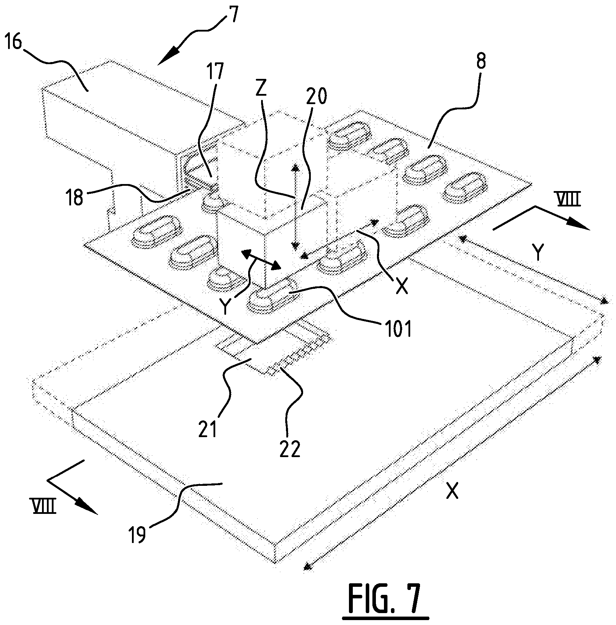

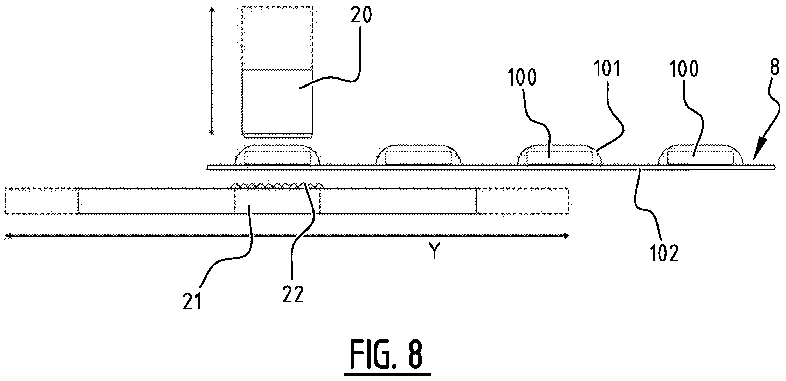

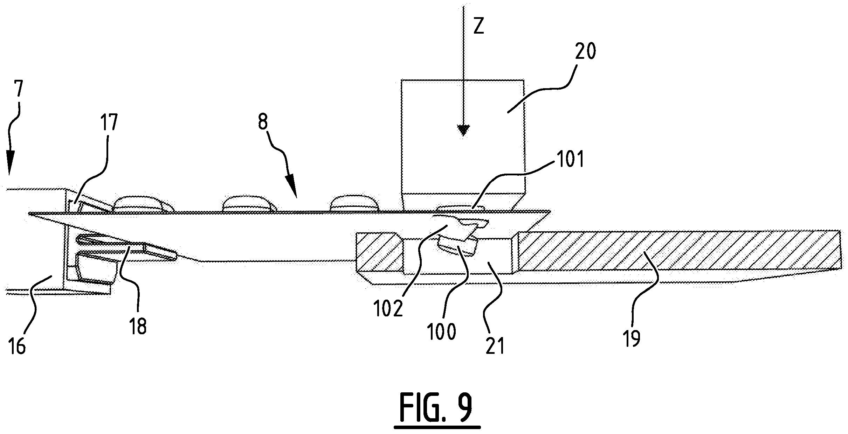

Also shown in figure 6 is a separating device 9 which is configured to press medicines selectively from a blister pack 8. This type of separating device 9 is shown in greater detail in figures 7-9. Separating device 9 comprises a first body 19 in the form of a platform 19 with passage 21 and a second body 20 in the form of clamping block 20 which is movable away from and toward platform 19 as indicated with arrow Z in figure 7. Both platform 19 and clamping block 20 are movable relative to blister pack 8 in a plane parallel to this blister pack 8. This is indicated schematically with arrows X and Y in figure 7. The separating device is configured to align clamping block 20 in X and Y direction with opening 21 so that, when clamping block 20 and platform 19 are moved toward each other (direction Z) with a holder 101 for a medicine 100 therebetween, this medicine 100 is pressed out of holder 101, see figure 9. Platform 19 and clamping block 20 are preferably movable together in the X-Y plane inside the separating device so that opening 21 and clamping block 20 are always aligned with each other in the X-Y plane. Passage 21 here receives the separated medicine 100.

Clamping block 20 and passage 21 of platform 19 must therefore be securely aligned with a holder 101 with the medicine 100 to be separated. As already noted above, it is important for this purpose that for each blister pack 8 the locations, for instance including dimensions, and state (full or empty) of each of the holders 101 in a blister pack 8 are known. Control unit 1000 can comprise for this purpose a database in which an overview of the configuration of holders 101 is stored for each type of medicine. During filling of storages 4 a blister pack can be presented to scanning device 53 which for instance reads and detects a unique identification number on the blister pack. On the basis of this number the correct configuration from the database can be linked to the blister pack. It is moreover possible for the scanning device to detect the positions and dimensions (as shown in figure 1A) for subsequent storage.

If control unit 1000 were for instance to determine that the supply of a specific medicine is (almost) finished, this can be indicated, for instance on screen 51 or via a remote system. The user can then fetch and present a new supply of medicines to scanning device 53. Apparatus 1, in particular control unit 1000, is preferably configured to detect whether the presented medicine corresponds to the type of medicine requested, for instance on the basis of a detected unique identification number on the presented blister pack 8. Once it has been ensured that the correct medicine is being presented, one of the storages 4 can be made available for filling. It is for instance possible here to envisage storages 4 being placed behind doors, wherein control unit 1000 is configured to lock and unlock the doors. The precise content, at least in respect of the type of medicine, per storage 4 is then known and is stored in memory 1000A of control unit 1000.

On the basis of the position data and states of holders 101 in the respective blister pack 8 in separating device 9 a holder 101 with medicine 100 is aligned with passage 21 and subsequently pressed out. The state of this holder 101 is then stored in memory 1000A, in the sense that what is stored is that this respective holder 101 in this respective blister pack 8 is empty.

Efficient pressing-out of medicines 100 takes place when a cutting device is provided close to an edge of passage 21, preferably in the form of serrated edge 22 which cuts into second layer 102 on the edge of the medicine for pressing out, whereby second layer 102 is easily split. Less force need be exerted here on medicine 100 by bodies 19 and 20, so that the risk of damage to medicine 100 is reduced. It can for instance be seen in figure 10 that cuts have been made in three edges of a holder 101. Following separation the medicine 100 drops into funnel 11 for further transport.

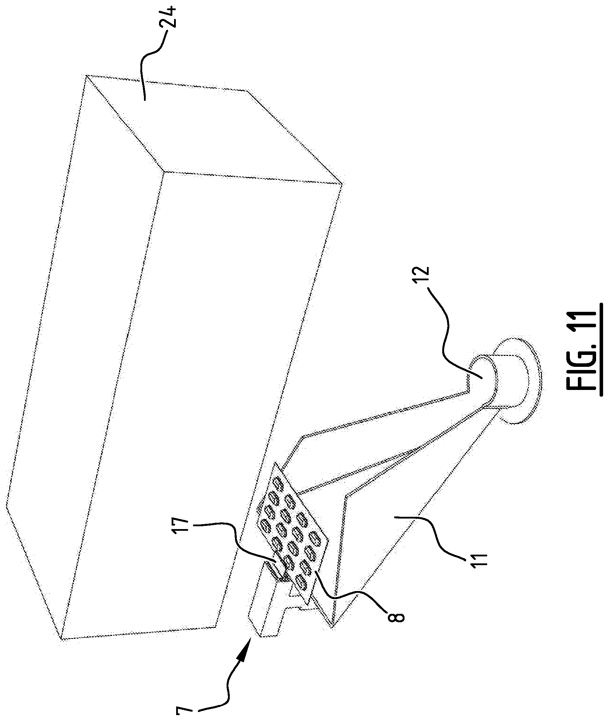

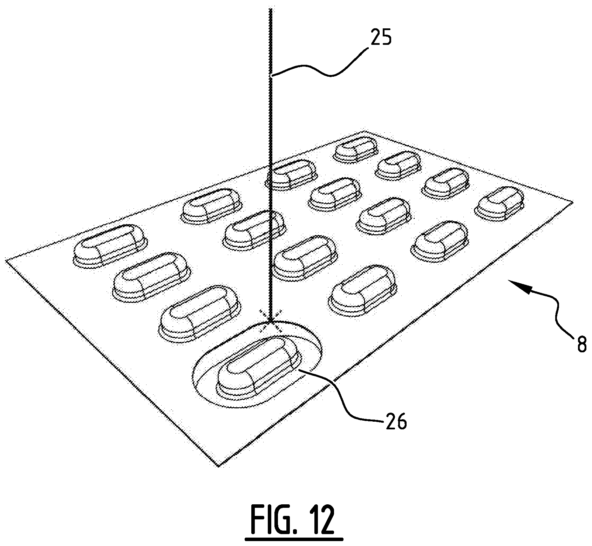

An alternative separating device is shown in figures 11 and 12. Where in the previous embodiment a medicine is separated from blister pack 8, in this embodiment a portion 26 of blister pack 8 with medicine is separated by being cut from the rest of blister pack 8. In this embodiment cutting takes place using a laser unit 24, wherein laser beam 25 is shown in figure 12. Laser beam 25 is movable, in per se known manner in a laser unit, in the plane parallel to the blister pack. Here too the separation takes place on the basis of the position data of holders 101 of blister pack 8. Since the position and dimensions of holders 101 are known, laser 25 can be controlled so as to move neatly round one or more holders 101 and thereby cut through the blister pack. The separated portion 26 will drop downward and enter funnel 11. Because, just as in the case of the pressing-out mechanism, the memory 1000A stores which part of the blister pack has already been cut out, the control unit controls laser device 24 to cut out a part of blister pack 8 which has not yet been cut away.

Alignment of holders 101 in a blister pack 8 for separation takes place subject to the stored position data of these holders 101 and the position of medicine holder 7. As stated, the relative position of medicine holder 7 and blister 8 is known, while the position of medicine holder 7 and conveyor belt 6 is also known. Through driving of conveyor belt 6 the position of blister 8, and thereby also the holders 101 thereof, is therefore known. The separation takes place on the basis hereof, for instance by aligning the bodies 20 and 19 or moving the laser 24. As alternative or as addition to this system, detection means can be provided for detecting the relative position of blister 8 and holders 101 thereof and separating device 9.

The construction of storages 4 will be discussed in greater detail with reference to figures 13 and 14. One storage 4 is filled with one type of medicine/blister pack 8, whereby different types of medicine can be accommodated in cabinets 3. As stated, control unit 1000 is configured to store the type of medicine per storage 4 in a memory 1000A. As shown best in figures 3 and 4, the plurality of grippers 7 on conveyor belt 6 are aligned with storages 4. Grippers 7 can then easily remove a new blister pack 8 from storages 4.

Referring to figure 13A, a storage comprises a first side 28 which can be provided with a door (not shown), whereby a number of blister packs can be placed in a centring device 27. Side 28 faces toward the outside of cabinets 3. Centring device 27 is configured to centre the held blister packs 8 in at least the X-direction. The opposite side 40 adjoins a transport system 5 and comprises an opening 31 for throughfeed of a blister pack (as also shown in figure 13B).

Centring device 27 further comprises a first 29 and second movable wall 30 which can move proportionally in opposite direction to each other in order to centre blister packs 8 in the X-direction when the centring device is activated. The position of blister pack 8 relative to a gripper 7 is then known. Provided for the purpose of transporting a blister pack 8 in the Y-direction so that it can be gripped by a gripper 7 is a transport system 34 for transporting a single blister pack 8 from storage cabinet 4 to gripper 7 (figure 14). Transport system 34 preferably comprises a transport element in the form of a wheel 32 which is driven by a drive element 33 and which engages on the lower surface of a blister pack and so feeds blister pack 8 through a passage opening 31. Blister pack 8 can now be clamped between first clamping element 17 and second clamping element 18.

When it is determined that a blister pack 8 is empty - the states of the different holders 101 in a blister pack 8 are after all tracked per blister pack 8 - the empty blister pack can be released by gripper 7 above a waste system (not shown). The empty blisters and perhaps other waste are discharged herein. Gripper 7 is then moved to a storage 4 in which the respective medicine is stored and a new blister pack 8 is picked up by gripper 7 in the above described manner.

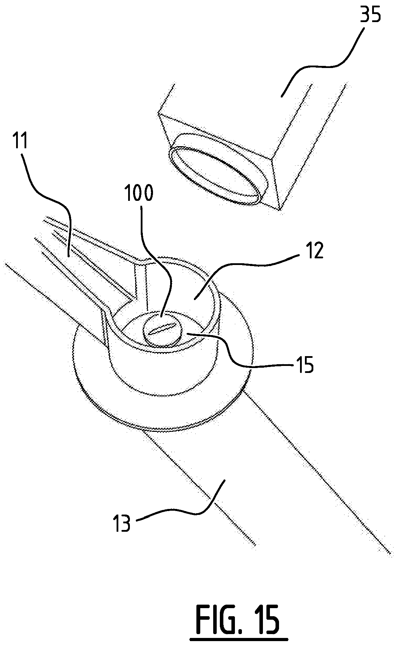

As already described above, buffering devices 12 are situated between funnels 11 and connection tubes 13. Such a buffer 12 is shown in greater detail in figure 15. A buffering device 12 is provided with a diaphragm 15 provided with a plurality of movable blades. In the closed situation as shown in figure 15 the medicines 100 are received (buffered), while when the blades are moved the diaphragm is thereby opened, the medicine 100 drops downward into connection tube 13. Also shown in figure 15 is a detection device in the form of a camera 35. Camera 35 makes recordings of the medicines on diaphragm 15, on the basis of which for instance control unit 1000 can determine whether the number and/or the type of medicine corresponds to the prescription. Should it for instance be determined that the number is not correct, the medicines can then be discharged in the case of an excess or be supplemented in the case of a shortfall. Through image recognition of medicines 100 the type can for instance also be determined and checked. Memory 1000A can comprise suitable comparison material for this purpose.

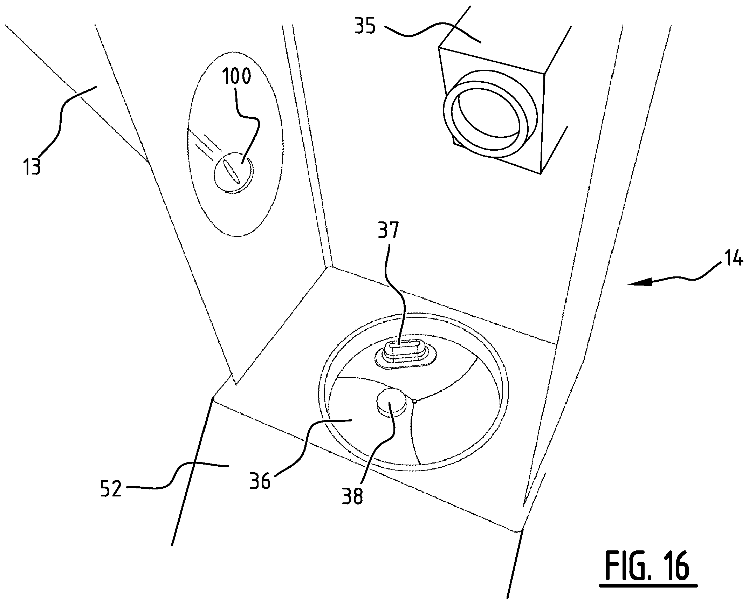

Connecting tubes 13 run out into the central collection tube 14 situated upstream of packaging device 52. In figure 16 can be seen that, following approval, the pressed-out medicine 100 is guided through connecting tube 13 to central collection device 14 where other medicines 36, 37, from for instance other tubes 13 or already separated earlier, are already present on a similar diaphragm 36. When all medicines for a packaging have been brought together, a detection device 35 once again checks whether the collected medicines correspond to the prescription. Following approval, diaphragm 36 opens and medicines 100, 37 and 38 are guided to packaging device 532 where the medicines are packaged. In figure 17 can be seen that the collected medicines 100, 37, 38 have been packed together in a foil packaging 39.

Figure 18 shows a processing diagram for packaging medicines. In a first step 1001 a prescription, designated schematically with 2000, is received for processing with the apparatus. The prescription 2000 comprises in this example a unique identification code 2001 for the patient and different unique codes 2003 for medicines which have been grouped per package 2002. In this example the first package must comprise three medicines (designated with 3x), the next one four (4x) and the last one only two (2x).

In a subsequent step 1002 the first medicine is selected from the list, in this example by carrying the correct medicine holder with the correct type of medicine to a separating device, and in a following step 1003 the correct number of this medicine are separated and buffered. A check is made in step 1004 as to whether the respective package 2002 has to comprise any more medicines. If so, steps 1002 and 1003 are repeated, in this example three times, for all medicines in the respective package.

Once all medicines of a single package have been selected and separated, the apparatus goes to step 1005 where the separated medicines for packaging are inspected and identified (see for instance figure 16). Alternatively or additionally hereto, a check can also be carried out after each separating step 1003 (see figure 15). When it is found in a step 1007 that the identified medicines do not correspond to the prescription, in particular the medicine in the first batch 2002, a procedure 1007A is carried out. This procedure can for instance entail the discharge of the medicines, wherein the schedule starts again from the beginning.

If the check is completed successfully, the separated medicines are packaged in a step 1006 in for instance a bag as shown in figure 17. In a step 1008 a check is then made as to whether there are still subsequent batches 2002 in this prescription. If this is the case, the procedure for selecting 1002 and selecting 1003 begins for the following batch 2002. The processing of the prescription for the patient is otherwise completed in step 1009 and a following prescription can be loaded in a step 1001.

The present invention is not limited to the shown embodiments but also extends to other embodiments falling within the scope of the appended claims.

## Claims

1. Apparatus (1) for packaging dosed quantities of solid medicines (100) on the basis of a predetermined prescription, comprising:
- a control unit (1000) for controlling the apparatus (1) and for receiving the prescription;
- a separating device (9) for separating at least one medicine (100) from a blister pack (8) subject to the received prescription; and
- a packaging device (52) configured to package the medicines (100) separated by the separating device (9),
**characterized in that** the apparatus (1) comprises a plurality of medicine holders (7), wherein a medicine holder (7) is configured to hold a blister pack (8) with medicines (100), wherein the medicine holders (7) are movable away from and toward the separating device (9) along a predetermined path, wherein the separating device (9) is disposed along this path and wherein the separating device (9) is configured to separate medicines (100) from a plurality of blister packs (8) held in the respective medicine holders (7) movable away from and toward the separating device (9).

2. Apparatus as claimed in claim 1, wherein a medicine holder (7) comprises a gripper which is configured to grip a blister pack (8).

3. Apparatus as claimed in claim 1 or 2, wherein the separating device (9) is disposed substantially above the packaging device (52).

4. Apparatus as claimed in at least one of the foregoing claims, also comprising an endless conveyor belt (6) provided with the plurality of medicine holders (7), wherein the separating device (9) is disposed along the path of the medicine holders (7), and a drive (61) for driving the endless conveyor belt (6) for the purpose of moving the medicine holders (7) along the separating device (9).

5. Apparatus as claimed in claims 3 and 4, comprising a plurality of separating devices (9) with medicine holders (7) associated therewith, wherein the separating devices (9) are disposed on a central, vertically extending collection tube (14) which debouches in the packaging device (52).

6. Apparatus as claimed in at least one of the foregoing claims, also comprising a housing in which at least the medicine holders (7) are located, wherein the housing is provided with a climate control system (70) for controlling the climate in the housing.

7. Apparatus as claimed in at least one of the foregoing claims, also comprising a buffering device (12) between a separating device (9) and the packaging device (52) for buffering separated medicines (100) for dispensing to the packaging device (52), wherein the buffering device (12) preferably comprises a diaphragm (15) provided with a plurality of movable blades.

8. Apparatus as claimed in claims 5 and 7, comprising a buffering device (12) between a separating device (9) and the central collection chute (14) for buffering separated medicines (100) for dispensing to the collection tube (14).

9. Apparatus as claimed in claim 7 or 8, also comprising a detection device (35) for inspecting and identifying the buffered medicine (100), wherein the control unit (1000) is configured to check the identified medicine (100) on the basis of the prescription.

10. Apparatus as claimed in at least one of the foregoing claims, wherein the control unit (1000) comprises a memory (1000A) which comprises position data, wherein the position data for the plurality of blister packs (8) comprise the positions of medicines (100) in the respective blister packs (8), wherein the separating device (9) is configured to separate at least one of the medicines (100) from the blister pack (8) on the basis of the position data of the respective blister pack (8), wherein the apparatus (1) preferably further comprises a detection device (35) for detecting the positions of the medicines (100) in a blister pack (8) for the purpose of providing the position data.

11. Apparatus as claimed in at least one of the foregoing claims, wherein the separating device (9) is configured to press at least one medicine (100) out of the blister pack (8), wherein the separating device (9) comprises a first body (19) and clamping body (20), wherein the separating device (9) is configured to press at least one medicine (100) out between the first body (19) and the clamping body (20), wherein the first body (19) is preferably provided with a milling device (22), more preferably in the form of serrations, for cutting into the blister pack (8).

12. Apparatus as claimed in at least one of the foregoing claims, wherein the apparatus (1) comprises at least one storage (3) for holding a plurality of blister packs (8), wherein the storage cabinet (3) comprises a centring device (27) for centring blister packs (8).

13. Method for packaging dosed quantities of solid medicines (100) on the basis of a predetermined prescription (2000), wherein the method comprises the steps of:
- providing a plurality of blister packs (8) with medicines (100) and held in medicine holders, wherein a medicine holder is configured to hold a blister pack (8);
- selecting (1002) at least one predetermined type of medicine (100) from the plurality of medicines (100) subject to the prescription (2000);
- separating (1003) a predetermined quantity of medicines (100) from the selected medicine (100) with a separating device (9); and
- packaging (1006) the separated medicines (100),
wherein the selection (1002) comprises of moving one of the medicine holders which holds a blister pack (8) with the predetermined type of medicine (100) to the separating device (9) along a predetermined path, wherein the separating device (9) is disposed along this path.

14. Method as claimed in claim 13, also comprising the step of buffering (1003) a set of separated medicine (100) for packaging in a single package prior to the step of packaging (1006).

15. Method as claimed in claim 14, further comprising the step of inspecting and identifying (1005) the buffered medicine (100) and checking the identified medicine (100) against the prescription (2000).

## Patentansprüche

1. Vorrichtung (1) zum Verpacken von dosierten Mengen fester Arzneimittel (100) auf der Grundlage eines vorbestimmten Rezepts, umfassend:
- eine Steuereinheit (1000) zum Steuern der Vorrichtung (1) und zum Empfangen des Rezepts;
- eine Trennvorrichtung (9) zum Trennen mindestens eines Medikaments (100) von einer Blisterverpackung (8) in Abhängigkeit von dem empfangenen Rezept; und
- eine Verpackungsvorrichtung (52), die dazu eingerichtet ist, die von der Trennvorrichtung (9) getrennten Medikamente (100) zu verpacken,
**dadurch gekennzeichnet, dass** die Vorrichtung (1) eine Vielzahl von Medikamentenhaltern (7) umfasst, wobei ein Medikamentenhalter (7) dazu eingerichtet ist, eine Blisterverpackung (8) mit Medikamenten (100) zu halten, wobei die Medikamentenhalter (7) entlang eines vorbestimmten Weges von der Trennvorrichtung (9) weg und zu ihr hin bewegbar sind, wobei die Trennvorrichtung (9) entlang dieses Weges angeordnet ist und wobei die Trennvorrichtung (9) dazu eingerichtet ist, Medikamente (100) von einer Vielzahl von Blisterverpackungen (8) zu trennen, die in den jeweiligen Medikamentenhaltern (7) gehalten werden, die von der Trennvorrichtung (9) weg und zu ihr hin bewegbar sind.

2. Vorrichtung nach Anspruch 1, wobei ein Medikamentenhalter (7) einen Greifer umfasst, der dazu eingerichtet ist, eine Blisterverpackung (8) zu greifen.

3. Vorrichtung nach Anspruch 1 oder 2, wobei die Trennvorrichtung (9) im Wesentlichen oberhalb der Verpackungsvorrichtung (52) angeordnet ist.

4. Vorrichtung nach mindestens einem der vorhergehenden Ansprüche, die auch ein endloses Förderband (6), das mit der Vielzahl von Medikamentenhaltern (7) versehen ist, wobei die Trennvorrichtung (9) entlang des Weges der Medikamentenhalter (7) angeordnet ist, und einen Antrieb (61) zum Antreiben des endlosen Förderbandes (6) zum Zwecke des Bewegens der Medikamentenhalter (7) entlang der Trennvorrichtung (9) umfasst.

5. Vorrichtung nach Anspruch 3 und 4, umfassend eine Mehrzahl von Trennvorrichtungen (9) mit diesen zugeordneten Medikamentenhaltern (7), wobei die Trennvorrichtungen (9) an einem zentralen, vertikal verlaufenden Sammelrohr (14) angeordnet sind, das in die Verpackungsvorrichtung (52) einmündet.

6. Vorrichtung nach mindestens einem der vorhergehenden Ansprüche, die auch ein Gehäuse umfasst, in dem mindestens die Medikamentenhalter (7) angeordnet sind, wobei das Gehäuse mit einem Klimasteuerungssystem (70) zur Steuerung des Klimas im Gehäuse versehen ist.

7. Vorrichtung nach mindestens einem der vorhergehenden Ansprüche, die auch eine Puffervorrichtung (12) zwischen einer Trennvorrichtung (9) und der Verpackungsvorrichtung (52) zum Puffern von getrennten Medikamenten (100) zur Abgabe an die Verpackungsvorrichtung (52) umfasst, wobei die Puffervorrichtung (12) vorzugsweise eine Membran (15) umfasst, die mit einer Vielzahl von beweglichen Klingen versehen ist.

8. Vorrichtung nach Anspruch 5 und 7, umfassend eine Puffervorrichtung (12) zwischen einer Trennvorrichtung (9) und der zentralen Sammelrutsche (14) zum Puffern der abgetrennten Medikamente (100) zur Abgabe an das Sammelrohr (14).

9. Vorrichtung nach Anspruch 7 oder 8, die auch eine Detektionsvorrichtung (35) zum Prüfen und Identifizieren des gepufferten Medikaments (100) umfasst, wobei die Steuereinheit (1000) dazu eingerichtet ist, das identifizierte Medikament (100) auf der Grundlage des Rezepts zu prüfen.

10. Vorrichtung nach mindestens einem der vorhergehenden Ansprüche, wobei die Steuereinheit (1000) einen Speicher (1000A) umfasst, der Positionsdaten umfasst, wobei die Positionsdaten für die Mehrzahl von Blisterverpackungen (8) die Positionen von Medikamenten (100) in den jeweiligen Blisterverpackungen (8) umfassen, wobei die Trennvorrichtung (9) dazu eingerichtet ist, mindestens eines der Medikamente (100) aus der Blisterverpackung (8) auf Basis der Positionsdaten der jeweiligen Blisterverpackung (8) zu trennen, wobei die Vorrichtung (1) vorzugsweise weiterhin eine Detektionsvorrichtung (35) zum Erfassen der Positionen der Medikamente (100) in einer Blisterverpackung (8) zum Zwecke der Bereitstellung der Positionsdaten umfasst.

11. Vorrichtung nach mindestens einem der vorangehenden Ansprüche, wobei die Trennvorrichtung (9) dazu eingerichtet ist, mindestens ein Medikament (100) aus der Blisterverpackung (8) herauszudrücken, wobei die Trennvorrichtung (9) einen ersten Körper (19) und einen Klemmkörper (20) umfasst, wobei die Trennvorrichtung (9) dazu eingerichtet ist, mindestens ein Medikament (100) zwischen dem ersten Körper (19) und dem Klemmkörper (20) herauszudrücken, wobei der erste Körper (19) vorzugsweise mit einer Fräsvorrichtung (22), besonders bevorzugt in Form von Zacken, zum Einschneiden in die Blisterverpackung (8) versehen ist.

12. Vorrichtung nach mindestens einem der vorhergehenden Ansprüche, wobei die Vorrichtung (1) mindestens ein Lager (3) zur Aufnahme einer Vielzahl von Blisterverpackungen (8) aufweist, wobei der Lagerschrank (3) eine Zentriervorrichtung (27) zur Zentrierung von Blisterverpackungen (8) aufweist.

13. Verfahren zum Verpacken von dosierten Mengen fester Medikamente (100) auf der Basis eines vorbestimmten Rezepts (2000), wobei das Verfahren die folgenden Schritte umfasst:
- Bereitstellen einer Vielzahl von Blisterverpackungen (8) mit Medikamenten (100), die in Medikamentenhaltern gehalten werden, wobei ein Medikamentenhalter zum Halten einer Blisterverpackung (8) eingerichtet ist;
- Auswählen (1002) mindestens einer vorbestimmten Art von Medikamenten (100) aus der Vielzahl von Medikamenten (100) in Abhängigkeit von dem Rezept (2000);
- Trennen (1003) einer vorbestimmten Menge von Medikamenten (100) von dem ausgewählten Medikament (100) mit einer Trennvorrichtung (9); und
- Verpacken (1006) der getrennten Medikamente (100),
wobei die Auswahl (1002) das Bewegen eines der Medikamentenhalter, der eine Blisterverpackung (8) mit der vorbestimmten Art von Medikamenten (100) hält, zu der Trennvorrichtung (9) entlang eines vorbestimmten Weges umfasst, wobei die Trennvorrichtung (9) entlang dieses Weges angeordnet ist.

14. Verfahren nach Anspruch 13, das auch den Schritt des Pufferns (1003) eines Satzes von getrennten Medikamenten (100) zum Verpacken in eine einzige Packung vor dem Schritt des Verpackens (1006) umfasst.

15. Verfahren nach Anspruch 14, das ferner den Schritt des Prüfens und Identifizierens (1005) des gepufferten Medikaments (100) und des Überprüfens des identifizierten Medikaments (100) anhand des Rezepts (2000) umfasst.

## Revendications

1. Dispositif (1) destiné à conditionner des quantités dosées de médicaments solides (100) sur la base d'une prescription prédéterminée, comprenant :
une unité de commande (1000) destinée à commander le dispositif (1) et à recevoir la prescription ;
un dispositif de séparation (9) destiné à séparer au moins un médicament (100) soumis à la prescription reçue par rapport à un emballage alvéolaire (8) ; et
un dispositif de conditionnement (52) configuré de manière à conditionner les médicaments (100) séparés par le dispositif de séparation (9),
**caractérisé en ce que** le dispositif (1) comprend une pluralité de supports de médicament (7), dans lequel un support de médicament (7) est configuré de manière à maintenir un emballage alvéolaire (8) avec des médicaments (100), dans lequel les supports de médicament (7) peuvent être écartés et rapprochés du dispositif de séparation (9) suivant un trajet prédéterminé, dans lequel le dispositif de séparation (9) est disposé le long de ce trajet et dans lequel le dispositif de séparation (9) est configuré de manière à séparer des médicaments (100) par rapport à une pluralité d'emballages alvéolaires (8) maintenus dans les supports de médicament (7) respectifs pouvant être écartés et rapprochés du dispositif de séparation (9).

2. Dispositif selon la revendication 1, dans lequel un support de médicament (7) comprend un élément de saisie qui est configuré de manière à saisir un emballage alvéolaire (8).

3. Dispositif selon la revendication 1 ou 2, dans lequel le dispositif de séparation (9) est disposé sensiblement au-dessus du dispositif de conditionnement (52).

4. Dispositif selon au moins l'une des revendications précédentes, comprenant, en outre, une bande de convoyeur sans fin (6) comportant la pluralité de supports de médicament (7), dans lequel le dispositif de séparation (9) est disposé le long du trajet des supports de médicament (7), et un dispositif d'entraînement (61) destiné à entraîner la bande de convoyeur sans fin (6) dans le but de déplacer les supports de médicament (7) le long du dispositif de séparation (9).

5. Dispositif selon les revendications 3 et 4, comprenant une pluralité de dispositifs de séparation (9), des supports de médicament (7) étant associés à ces derniers, dans lequel les dispositifs de séparation (9) sont disposés sur un tube de collecte central s'étendant verticalement (14) qui débouche dans le dispositif de conditionnement (52).

6. Dispositif selon au moins l'une des revendications précédentes, comprenant, en outre, un logement dans lequel au moins les supports de médicament (7) sont agencés, dans lequel le logement comporte un dispositif de commande de température (70) destiné à commander la température dans le logement.

7. Dispositif selon au moins l'une des revendications précédentes, comprenant, en outre, un dispositif tampon (12) entre un dispositif de séparation (9) et le dispositif de conditionnement (52) afin de mettre en tampon des médicaments (100) séparés à distribuer au dispositif de conditionnement (52), dans lequel le dispositif tampon (12) comprend, de préférence, un diaphragme (15) comportant une pluralité de lames mobiles.

8. Dispositif selon les revendications 5 et 7, comprenant un dispositif tampon (12) entre un dispositif de séparation (9) et une goulotte de collecte centrale (14) afin de mettre en tampon des médicaments (100) séparés à distribuer au tube de collecte (14).

9. Dispositif selon la revendication 7 ou 8, comprenant, en outre, un dispositif de détection (35) destiné à inspecter et à identifier le médicament (100) mis en tampon, dans lequel l'unité de commande (1000) est configurée de manière à contrôler le médicament (100) identifié sur la base de la prescription.

10. Dispositif selon au moins l'une des revendications précédentes, dans lequel l'unité de commande (1000) comprend une mémoire (1000A) qui comprend des données de position, dans lequel les données de position de la pluralité d'emballages alvéolaires (8) comprennent les positions des médicaments (100) dans les emballages alvéolaires (8) respectifs, dans lequel le dispositif de séparation (9) est configuré de manière à séparer au moins l'un des médicaments (100) de l'emballage alvéolaire (8) sur la base des données de position de l'emballage alvéolaire (8) respectif, dans lequel le dispositif (1) comprend, en outre, de préférence, un dispositif de détection (35) destiné à détecter les positions des médicaments (100) dans un emballage alvéolaire (8) dans le but de fournir les données de position.

11. Dispositif selon au moins l'une des revendications précédentes, dans lequel le dispositif de séparation (9) est configuré de manière à presser au moins un médicament (100) hors de l'emballage alvéolaire (8), dans lequel le dispositif de séparation (9) comprend un premier corps (19) et un corps de retenue (20), dans lequel le dispositif de séparation (9) est configuré de manière à presser au moins un médicament (100) vers l'extérieur entre le premier corps (19) et le corps de retenue (20), dans lequel le premier corps (19) comporte, de préférence, un dispositif de fraisage (22), plus préférablement, sous forme de dentelure, afin de d'usiner l'emballage alvéolaire (8).

12. Dispositif selon au moins l'une des revendications précédentes, dans lequel le dispositif (1) comprend au moins un compartiment de stockage (3) destiné à contenir une pluralité d'emballages alvéolaires (8), dans lequel le coffret de stockage (3) comprend un dispositif de centrage (27) destiné à centrer les emballages alvéolaires (8).

13. Procédé de conditionnement de quantités dosées de médicaments solides (100) sur la base d'une prescription prédéterminée (2000), dans lequel le procédé comprend les étapes de :
introduction de médicaments (100) dans une pluralité d'emballages alvéolaires (8) et maintien dans des supports de médicament, dans lequel un support de médicament est configuré de manière à maintenir un emballage alvéolaire (8) ;
sélection (1002) d'au moins un type de médicament (100) prédéterminé à partir de la pluralité de médicaments (100) soumis à la prescription (2000) ;
séparation (1003) d'une quantité de médicaments (100) prédéterminée à partir du médicament (100) sélectionné avec un dispositif de séparation (9) ; et
conditionnement (1006) des médicaments (100) séparés,
dans lequel la sélection (1002) comprend le déplacement d'un des supports de médicament qui maintient un emballage alvéolaire (8) avec le type de médicament (100) prédéterminé sur le dispositif de séparation (9) le long d'un trajet prédéterminé, dans lequel le dispositif de séparation (9) est disposé le long de ce trajet.

14. Procédé selon la revendication 13, comprenant, en outre, l'étape de mise en tampon (1003) d'un jeu de médicaments (100) séparés à conditionner dans un emballage unique avant l'étape de conditionnement (1006).

15. Procédé selon la revendication 14, comprenant, en outre, l'étape d'inspection et d'identification (1005) du médicament (100) mis en tampon et de contrôle du médicament (100) identifié par rapport à la prescription (2000).
